# EUROPÄISCHE PATENTANMELDUNG

(11) **EP 0 752 426 A2**
(43) Veröffentlichungstag der Anmeldung: **08.01.1997**
(21) Anmeldenummer: 96110193.8
(22) Anmeldetag: 24.06.1996
(51) Int. Cl.: C07K 14/47, C07K 17/02, C07K 17/08, G01N 33/53

(54) **Synthetische Kalibratoren für den Einsatz in Immunoassays, bestehend aus Analyten oder Teilsequenzen davon, die an inerte Trägermoleküle konjugiert sind**

(30) Priorität: 06.07.1995 DE 19524572
(71) Anmelder: BAYER AG, 51368 Leverkusen (DE)
(72) Erfinder: Doth, Margit, Dr., 47800 Krefeld (DE); Petry, Christoph, Dr., 47800 Krefeld (DE)

(57) **Zusammenfassung**

Die Erfindung betrifft synthetische Kalibratoren für immunologische Teste, wobei für den Analyten spezifische Epitope an andere Proteine oder an synthetische Träger gekoppelt werden.

## Beschreibung

Zum Nachweis von Proteinen in Serum- oder Urinproben für medizinischdiagnostische Zwecke werden häufig wegen besonders guter Spezifität und Sensitivität Immunoassays eingesetzt. Dazu bedarf es neben einem oder zwei spezifischen Antikörpern eines Kalibrators, der als Vergleichsmaßstab zur Quantifizierung der Patientenproben und als Positivkontrolle benutzt wird. Besonders für automatisierte Assays in großen Analyselaboratorien ist die Lagerung der Kalibratoren bei 4°C für mehrere Wochen bis Monate wünschenswert. Diese Anforderungen an die Stabilität der Kalibratorformulierung können je nach Analyt Schwierigkeiten bereiten, wenn z.B. die Löslichkeit unter physiologischen Salz- und pH-Bedingungen nicht gewährleistet ist. Als Beispiel seien hier Troponin I und Troponin T genannt, die nur in denaturierenden Lösungen (6 M Harnstoff, 0,01 M Dithiothreitol) hinreichend stabil und löslich sind. Mit dieser denaturierenden Formulierung kann jedoch kein Immunoassay etabliert werden, da die Antikörper unter dieser Behandlung leiden.

Es ist bekannt, daß Proteine in Lösung wenig stabil sind und daß sie enthaltende Reagenzien häufig in gefriergetrockneter Form vertrieben werden und mit einem Lösungsmittel geeigneter Zusammensetzung vor der Verwendung von dem Benutzer aufgelöst werden müssen. Wenn man die auf diese Weise erhaltenen Lösungen bei 4°C aufbewahrt, kann man sie während mehrerer Tage benutzen, selbst wenn die tägliche Bestimmung eine gewisse Veränderung der Konzentration des Reagens zeigt. So werden im allgemeinen - und es wird auch im Fall von Troponin I und Troponin T empfohlen - die ausgehend von dem gefriergetrockneten Material erhaltenen Vergleichslösungen für längere Lagerung in Einheitsdosisform einzufrieren.

Eine Möglichkeit für die Entwicklung eines stabilen Kalibrators besteht darin, das gesamte Protein oder Teilsequenzen an ein Trägermolekül zu konjugieren und so die Löslichkeit und/oder Stabilität zu erhöhen. Das Trägermolekül sollte dabei immunologisch nicht reaktiv, aber chemisch konjugationsfähig sein.

Die vorliegende Erfindung betrifft einen synthetischen Kalibrator mit sehr guter Stabilität für den Einsatz in Immunoassays zur Bestimmung medizinisch relevanter Analyten in Blut-, Plasma-, Serum- oder Urinproben. Die Herstellung beinhaltet die chemische Kopplung des Analyten oder seiner Teilsequenzen an immunologisch inerte konjugierbare Trägermoleküle. Die künstliche Kalibratorsubstanz liegt in physiologischen Puffern mit Salzkonzentrationen von 0,01 -5 M, insbesondere 0,05 - 0,5 M und pH-Bedingungen zwischen pH 2 und 12, insbesondere pH 6 - 8 vor. Die Puffer können Konservierungsmittel wie z.B. Natriumazid, Proclin 150, Stabilisierungsmittel wie z.B. Rinderserumalbumin (BSA) Human- oder Mausserum, Mausimmunglobuline, andere Zusätze wie z.B. β-Mercaptoethanol, EDTA, oder Detergenzien wie z.B. Tween 20, Natriumdodecylsulfat (SDS), Triton X 100 enthalten.

Die chemische Konjugation verläuft nach bekannten Methoden, die in der Literatur (S.S.Wong, Chemistry of protein conjugation and cross-linking, 1991 CRC Press Inc.) beschrieben sind.

Cross-linking Reagenzien können homobifunktional oder heterobifunktional sein. Die ersten reagieren mit Aminogruppen wie z.B. Glutaraldehyd mit der ε-Aminogruppe des Lysin, mit Sulfhydrylgruppen wie z.B. Bismaleimide oder alkylierende Agenzien, mit Carboxylgruppen wie z.B. Carbodiimide in Kombination mit Diaminen oder mit Phenolat- und Imidazolylgruppen wie z.B. Diazoniumsalze, die mit aromatischen Aminosäuren wie Tyrosin oder Histidin reagieren. Weiterhin kommen heterobifunktionale Cross-linker für eine Kopplung von Peptiden an Trägermoleküle in Frage. Mit Aminogruppen einerseits und Sulfhydrylgruppen andererseits reagiert z.B. N-Succinimidyl-4-(N-maleimidomethyl)-cyclohexan-1-carboxylat (SMCC). Mit Carboxyl- und Sulfhydryl- oder Aminogruppen reagieren z.B. Pyridyl-2,2'-dithiobenzyldiazoacetat (PDD) oder p-Nitrophenyldiazoacetat. Ein Carbonyl- und Sulfhydrylgruppen bindendes Reagenz ist z.B. 1-(Aminooxy)-4-{(3-nitro-2-pyridyl)dithio}butan. Sind in den fraglichen Proteinen, Peptiden oder im Trägermolekül keine Sulfhydrylgruppen vorhanden, können diese über 2-Iminothiolan-hydrochlorid (2-IT) oder ähnliche Reagenzien an Aminogruppen z.B. des Lysins angefügt werden.

Das Trägermolekül kann bestehen aus Proteinen oder Polymeren, die konjugierbare Gruppen enthalten. Als Trägermolekül kommen vorzugsweise große Proteine (MW > 100 kD) in Frage, wie z.B. Ferritin, α₁-Macroglobulin, Thyroglobulin, bei denen im Fall eines Sandwich-Assays keine sterische Behinderung der beiden Antikörper zu erwarten ist. Es lassen sich jedoch auch kleinere Proteine wie z.B. BSA als Trägermaterial benutzen. Zur Kopplung geeignete reaktive Gruppen in Proteinen sind z.B. Aminogruppen der Seitenketten z.B. in Lysin oder die Sulfhydrylgruppe des Cysteins.

Als synthetische Trägermaterialien kommen Polymere wie z.B. Polyvinylalkohole, Polyacrylate, Polysulfonate, aber auch Polyamide, Polyester und Polyether in Frage. Die Kopplung von Peptiden an diese Verbindungen erfolgt über die entsprechenden funktionellen Gruppen (Hydroxyl-, Carboxyl- oder Aminogruppen).

Weiterhin können neben Proteinen Naturstoffe wie Chitin als auch Chitosan oder Gelatine als Trägermaterialien dienen.

Die zu konjugierende Substanz umfaßt medizinisch-diagnostisch relevante Proteine, die entweder als Ganzes oder als Teilsequenzen an den Träger gebunden werden. Als Teilsequenzen kommen durch Proteasespaltung entstehende Fragmente oder synthetische Peptide des Ursprungsmoleküls, die mit Hilfe handelsüblicher Synthesizer produziert werden, in Frage. Dabei kann die Anzahl der verschiedenen Sequenzen zwischen 1 und 20 liegen. Für einen Sandwich-Assay, d.h. unter Benutzung zweier für den Analyten spezifischer Antikörper, müssen ein ausreichend langes oder besser zwei verschiedene Peptide an den Träger gebunden werden.

Die zu konjugierenden synthetischen Peptide können durch Modifikation der Ausgangs(Analyt)sequenz z.B. Anfügen eines Cysteins oder Lysins eine leichtere Kopplung ermöglichen. Die Peptide sollten Epitope für die Analyt-spezifischen Antikörper umfassen; das sind in der Regel Proteinsequenzen aus der Moleküloberfläche.

Die Erfindung betrifft insbesondere ein synthetisches Kalibratormaterial für das cardiale Troponin I, ein herzspezifisches Protein, das bei der Diagnostik des akuten Myokardinfarktes eine Rolle spielt. Der Kalibrator besteht aus zwei künstlichen Peptiden dieses Analyten, die an BSA als Träger konjugiert wurden.

### Herstellung eines erfindungsgemäßen Kalibrators

Als Trägermolekül wurde Rinderserumalbumin gewählt, die Peptide des humanen cardialen TnI hatten folgende Sequenzen:
- Peptid 1:: Aminosäuren 27-40 der TnI-Sequenz und
- Peptid 2:: Aminosäuren 69-85 der TnI-Sequenz

Am Peptid 1 wurde C-terminal ein Cystein, an Peptid 2 Alanin und Lysin zur Erleichterung der Konjugation angehängt. Zur Kopplung wurde eine für die Proteinkonjugation bekannte Methode gewählt (S. Yoshitake et al., Eur. J. Biochem., 101, 395, 1979). Das Trägerprotein BSA wurde mit SMCC aktiviert, indem 20 mM SMCC in DMF gelöst und als 25-facher Überschuß zum BSA gegeben wurde. Die Mischung inkubierte 25 min bei 25°C. Beendet wurde die Reaktion durch Zugabe von 1 µm Glycinlösung (10 min, 25°C). Die Peptide wurden entweder über die Sulfhydrylgruppe in ihrer Sequenz oder durch Einfügen einer solchen mit 2-IT an das aktivierte BSA gebunden.

Zur Reinigung der Kalibratorsubstanz, d.h. Abtrennung der niedermolekularen Ausgangssubstanzen wurde ein Gelchromatographie (Superdex 200) durchgeführt. Anschließend erfolgte UV-spektrometrischen Konzentrationsbestimmung und Stabilisierung über 0,5 % BSA / 0,1 % Natriumazid. Möglich ist weiterhin eine affinitätschromatographische Reinigung über die sequenzspezifischen Antikörper, die auch im Immunoassay eingesetzt werden, oder jede andere übliche Methode zur Proteinreinigung.

### Durchführung der Immunoassays

### 1. ELISA

Mit dem oben genannten erfindungsgemäßen Kalibrator wurde nach gängigen Methoden ein Sandwich-ELISA durchgeführt, der zeigt, daß die Kalibratorsubstanz als Ersatz für den natürlichen Analyten eingesetzt werden kann.

Auf Mikrotiterplatten (Greiner) wurde ein Antikörper gegen die Sequenz 1 des TnI, die an den künstlichen Kalibrator konjugiert wurde, aufgebracht.

Anschließend erfolgte die Inkubation mit dem Kalibrator. Als Detektionsantikörper wurde ein in Ziegen produzierter Antikörper gegen das 2. Peptid des TnI, das an den künstlichen Kalibrator gekoppelt war benutzt. Die Bindung dieses Antikörpers an den künstlichen Kalibrator wurde nachgewiesen über einen anti-Ziege-IgG Antikörper, der mit alkalischer Phosphatase konjugiert war. Dieses Enzym katalysiert Farbreaktionen, deren Intensität proportional zur Menge des im Sandwich gebundenen Analyten ist. Mit diesem ELISA konnte gezeigt werden, daß das Konjugat mindestens 24 h bei 37°C stabil ist.

### 2. Automatisierter Sandwich-Assay

Der künstliche Kalibrator wurde auf dem automatisierten Analyser Immuno 1 (Miles Diagnostics) eingesetzt. Das Assay-Format war ebenfalls ein Sandwich unter Benutzung der gleichen Antikörper wie im ELISA unter 1. beschrieben. Der erste Antikörper des Sandwich bindet Sequenz 1 auf dem künstlichen Kalibrator. Der Antikörper ist mit FITC markiert und wird über anti-FITC auf Magnetpartikeln immobilisiert. Der 2. Antikörper des Sandwich trägt alkalische Phosphatase und katalysiert die Farbreaktion. Die Antikörper-Inkubation erfolgte sequentiell. Auch bei diesem Testverfahren konnte eine der Konzentration der Kalibratorsubstanz proportionale Zunahme der Farbintensität gezeigt werden.

## Patentansprüche

1. Synthetischer Kalibrator für Immunoassays, bestehend aus einem Trägermolekül und daran gekoppelt ein oder mehrere für den Analyten spezifische Epitope.

2. Synthetischer Kalibrator gemäß Anspruch 1, worin das Trägermolekül ein Protein oder ein Polymer ist.

3. Synthetischer Kalibrator gemäß Anspruch 2, worin das Trägermolekül Rinderserumalbumin, Ferritin, α₁-Macroglobulin, Thyroglobulin oder ein Polyvinylalkohol, Polyacrylat, Polysulfonat, Polyamid, Polyester oder Polyether ist.

4. Synthetischer Kalibrator gemäß den Ansprüchen 1 bis 3, enthaltend als Epitope Peptide des humanen cardialen Troponin I.

5. Synthetischer Kalibrator gemäß den Anspruch 4, enthaltend die Peptide mit den Aminosäuren 27-40 und 69-85 des humanen cardialen Troponin I.

6. Immunologischer Test, enthaltend den Kalibrator gemäß den Ansprüchen 1 bis 5.
